(19) Europäisches Patentamt
European Patent Office
Office européen des brevets

(11) EP 2 272 989 B1

(12) **EUROPEAN PATENT SPECIFICATION**

(45) Date of publication and mention of the grant of the patent:
**04.01.2017 Bulletin 2017/01**

(51) Int Cl.:
***C12Q 1/68*** *(2006.01)*

(21) Application number: **10251223.3**

(22) Date of filing: **08.07.2010**

(54) **Methods and reagents for the early detection of melanoma**

Verfahren und Reagenzien zur frühen Erkennung von Melanomen

Procédés et réactifs pour la détection précoce du mélanome

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **08.07.2009 US 223894 P**

(43) Date of publication of application:
**12.01.2011 Bulletin 2011/02**

(73) Proprietor: **Janssen Diagnostics, LLC**
**Raritan, NJ 08869 (US)**

(72) Inventors:
- **Talantov, Dmitri**
  **San Diego, CA 92130 (US)**
- **Palma, John F.**
  **Carlsbad, CA 92009 (US)**
- **Jatkoe, Tim**
  **San Diego, CA 92122 (US)**
- **Vener, Tatiana**
  **Stirling, NJ 07980 (US)**
- **Wang, Yixin**
  **Basking Ridge,NJ 07920 (US)**
- **Wang, Haiying**
  **Bridgewater, NJ 08807 (US)**

(74) Representative: **Carpmaels & Ransford LLP**
**One Southampton Row**
**London WC1B 5HA (GB)**

(56) References cited:
**WO-A2-2006/002433**

- **KAUFFMAN L ET AL: "GENE EXPRESSION MARKERS TO FACILITATE MELANOMA DIAGNOSIS IN SKIN BIOPSIES" JOURNAL OF CUTANEOUS PATHOLOGY, vol. 36, no. 1, January 2009 (2009-01), page 110, XP002609009 & 45TH ANNUAL MEETING OF THE AMERICAN-SOCIETY-OF-DERMATOPATHOLO GY; SAN FRANCISCO, CA, USA; OCTOBER 16 -19, 2008 ISSN: 0303-6987**
- **ARENBERGER P ET AL: "Quantitative detection of circulating melanoma cells by real time RT-PCR in patients with malignant melanoma" JOURNAL OF INVESTIGATIVE DERMATOLOGY, vol. 125, no. 6, December 2005 (2005-12), page A31, XP002609010 & 2ND ANNUAL MEETING OF THE AUSTRALASIAN-SOCIETY-FOR-DERMATOLOG Y-RESEA RCH; PERTH, AUSTRALIA; 20050514, ISSN: 0022-202X**
- **MCMASTERS KELLY M ET AL: "Lessons learned from the Sunbelt Melanoma Trial." JOURNAL OF SURGICAL ONCOLOGY 1 JUL 2004 LNKD- PUBMED:15221928, vol. 86, no. 4, 1 July 2004 (2004-07-01), pages 212-223, XP002609011 ISSN: 0022-4790**
- **ROTHBERG BONNIE E GOULD ET AL: "Nuclear to non-nuclear Pmel17/gp100 expression (HMB45 staining) as a discriminator between benign and malignant melanocytic lesions." MODERN PATHOLOGY : AN OFFICIAL JOURNAL OF THE UNITED STATES AND CANADIAN ACADEMY OF PATHOLOGY, INC SEP 2008 LNKD- PUBMED:18552823, vol. 21, no. 9, September 2008 (2008-09), pages 1121-1129, XP002609012 ISSN: 1530-0285**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ADEMA GOSSE J ET AL: "Molecular Characterization of the melanocyte Lineage-specific Antigen gp100" JOURNAL OF BIOLOGICAL CHEMISTRY, AMERICAN SOCIETY FOR BIOCHEMISTRY AND MOLECULAR BIOLOGY, INC, US, vol. 269, no. 31, 1 August 1994 (1994-08-01), pages 20126-20133, XP002188007 ISSN: 0021-9258
- WAGNER S N ET AL: "Analysis of Pmel17/gp100 expression in primary human tissue specimens: implications for melanoma immuno- and gene-therapy." CANCER IMMUNOLOGY, IMMUNOTHERAPY : CII JUN 1997 LNKD- PUBMED:9222283, vol. 44, no. 4, June 1997 (1997-06), pages 239-247, XP002609013 ISSN: 0340-7004
- SCHAEFER K -L ET AL: "Expression profiling of malignant melanoma of soft parts" PATHOLOGY RESEARCH AND PRACTICE, vol. 198, no. 3, 2002, page 208, XP002609014 & 86TH MEETING OF THE GERMAN SOCIETY OF PATHOLOGY; VIENNA, AUSTRIA; APRIL 03-06, 2002 ISSN: 0344-0338
- BROUWENSTIJN N ET AL: "Transcription of the gene encoding melanoma-associated antigen gp100 in tissues and cell lines other than those of the melanocytic lineage" BRITISH JOURNAL OF CANCER, NATURE PUBLISHING GROUP, LONDON, GB, vol. 76, no. 12, 1 January 1997 (1997-01-01), pages 1562-1566, XP002587572 ISSN: 0007-0920
- SCHREURS M W J ET AL: "GENETIC VACCINATION AGAINST THE MELANOCYTE LINEAGE-SPECIFIC ANTIGEN GP100 INDUCES CYTOTOXIC LYMPHOCYTE-MEDIATED TUMOR PROTECTION" CANCER RESEARCH, AMERICAN ASSOCIATION FOR CANCER RERERACH, US, vol. 58, no. 12, 1 January 1998 (1998-01-01), pages 2509-2514, XP000919304 ISSN: 0008-5472
- KASHANI-SABET MOHAMMED ET AL: "A multi-marker assay to distinguish malignant melanomas from benign nevi" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0901185106, vol. 106, no. 15, 14 April 2009 (2009-04-14), pages 6268-6272, XP002562659 ISSN: 0027-8424 [retrieved on 2009-03-30]
- KOH STEPHEN S ET AL: "Molecular classification of melanomas and nevi using gene expression microarray signatures and formalin-fixed and paraffin-embedded tissue." MODERN PATHOLOGY : AN OFFICIAL JOURNAL OF THE UNITED STATES AND CANADIAN ACADEMY OF PATHOLOGY, INC APR 2009 LNKD- PUBMED:19270649, vol. 22, no. 4, April 2009 (2009-04), pages 538-546, XP002609015 ISSN: 1530-0285
- ALEXANDRESCU DORU T ET AL: "Melanoma-specific marker expression in skin biopsy tissues as a tool to facilitate melanoma diagnosis." THE JOURNAL OF INVESTIGATIVE DERMATOLOGY JUL 2010 LNKD- PUBMED:20357814, vol. 130, no. 7, July 2010 (2010-07), pages 1887-1892, XP002609016 ISSN: 1523-1747
- TALANTOV DMITRI ET AL: "Novel genes associated with malignant melanoma but not benign melanocytic lesions", CLINICAL CANCER RESEARCH, THE AMERICAN ASSOCIATION FOR CANCER RESEARCH, US, vol. 11, no. 20, 15 October 2005 (2005-10-15), pages 7234-7242, XP002377436, ISSN: 1078-0432, DOI: 10.1158/1078-0432.CCR-05-0683

SEQUENCE LISTING

<110> VERIDEX, LLC

<120> METHODS AND REAGENTS FOR THE EARLY DETECTION OF MELANOMA

<130> P055082EP

<150> uS61/223,894
<151> 2009-07-08

<160> 17

<170> seqwin99, version 1.02

<210> 1
<211> 20
<212> DNA
<213> Artificial sequence

<220>
<223> Primer

<400> 1
agcttatcat gcctggtcaa 20

<210> 2
<211> 20
<212> DNA
<213> Primer

<400> 2
gggtacggag aagtcttgct 20

<210> 3
<211> 21
<212> DNA
<213> Artificial Sequence

<220>
<223> Probe

<220>
<221> misc_feature
<222> 1
<223> N = A labelled with carboxyfluorescein

<220>
<221> misc_feature
<222> 21
<223> N = T labelled with 2,5-di-tert-butylhydroquinone-1

<400> 3
nggttccgct atcgtgggca n 21

<210> 4
<211> 2082
<212> DNA
<213> Homo Sapiens

<400> 4

```
atcactgtag tagtagctgg aaagagaaat ctgtgactcc aattagccag ttcctgcaga    60
ccttgtgagg actagaggaa gaatgctcct ggctgttttg tactgcctgc tgtggagttt   120
ccagacctcc gctggccatt tccctagagc ctgtgtctcc tctaagaacc tgatggagaa   180
ggaatgctgt ccaccgtgga gcggggacag gagtccctgt ggccagcttt caggcagagg   240
ttcctgtcag aatatccttc tgtccaatgc accacttggg cctcaatttc ccttcacagg   300
ggtggatgac cgggagtcgt ggccttccgt cttttataat aggacctgcc agtgctctgg   360
caacttcatg ggattcaact gtggaaactg caagtttggc ttttggggac caaactgcac   420
```

```
agagagacga ctcttggtga gaagaaacat cttcgatttg agtgccccag agaaggacaa   480
atttttgcc tacctcactt tagcaaagca taccatcagc tcagactatg tcatccccat   540
agggacctat ggccaaatga aaaatggatc aacacccatg tttaacgaca tcaatattta   600
tgacctcttt gtctggatgc attattatgt gtcaatggat gcactgcttg ggggatctga   660
aatctggaga gacattgatt ttgcccatga agcaccagct tttctgcctt ggcatagact   720
cttcttgttg cggtgggaac aagaaatcca gaagctgaca ggagatgaaa acttcactat   780
tccatattgg gactggcggg atgcagaaaa gtgtgacatt tgcacagatg agtacatggg   840
aggtcagcac cccacaaatc ctaacttact cagcccagca tcattcttct cctcttggca   900
gattgtctgt agccgattgg aggagtacaa cagccatcag tctttatgca atggaacgcc   960
cgagggacct ttacggcgta atcctggaaa ccatgacaaa tccagaaccc caaggctccc   1020
ctcttcagct gatgtagaat tttgcctgag tttgacccaa tatgaatctg gttccatgga   1080
taaagctgcc aatttcagct ttagaaatac actggaagga tttgctagtc cacttactgg   1140
gatagcggat gcctctcaaa gcagcatgca caatgccttg cacatctata tgaatggaac   1200
aatgtcccag gtacagggat ctgccaacga tcctatcttc cttcttcacc atgcatttgt   1260
tgacagtatt tttgagcagt ggctccgaag gcaccgtcct cttcaagaag tttatccaga   1320
agccaatgca cccattggac ataaccggga atcctacatg gttcctttta taccactgta   1380
cagaaatggt gatttcttta tttcatccaa agatctgggc tatgactata gctatctaca   1440
agattcagac ccagactctt ttcaagacta cattaagtcc tatttggaac aagcgagtcg   1500
gatctggtca tggctccttg gggcggcgat ggtaggggcc gtcctcactg ccctgctggc   1560
agggcttgtg agcttgctgt gtcgtcacaa gagaaagcag cttcctgaag aaaagcagcc   1620
actcctcatg gagaaagagg attaccacag cttgtatcag agccatttat aaaaggctta   1680
ggcaatagag tagggccaaa aagcctgacc tcactctaac tcaaagtaat gtccaggttc   1740
ccagagaata tctgctggta tttttctgta aagaccattt gcaaaattgt aacctaatac   1800
aaagtgtagc cttcttccaa ctcaggtaga acacacctgt ctttgtcttg ctgttttcac   1860
tcagcccttt taacattttc ccctaagccc atatgtctaa ggaaaggatg ctatttggta   1920
atgaggaact gttatttgta tgtgaattaa agtgctctta ttttaaaaaa ttgaaataat   1980
tttgattttt gccttctgat tatttaaaga tctatatatg ttttattggc cccttcttta   2040
ttttaataaa acagtgagaa atctaaaaaa aaaaaaaaaa aa                      2082
```

<210> 5
<211> 21
<212> DNA
<213> Artificial Sequence

<220>
<223> Primer

<400> 5
aggttccgct atcgtgggca t 21

<210> 6
<211> 17
<212> DNA
<213> Artificial sequence

<220>
<223> Primer

<400> 6
cggcccgaga gatacgc 17

<210> 7
<211> 13
<212> DNA
<213> Artificial Sequence

<220>
<223> Probe

<220>
<221> misc_feature

<222> 1
<223> N = A labelled with carboxyfluorescein

<400> 7
ntccggcggc cac 13

<210> 8
<211> 23
<212> DNA
<213> Artificial sequence

<220>
<223> Primer

<400> 8
actatggcct tgtctgggat ctc 23

<210> 9
<211> 25
<212> DNA
<213> Artificial Sequence

<220>
<223> Primer

<400> 9
agatatggaa cctgaagttg cactg 25

<210> 10
<211> 18
<212> DNA
<213> Artificial sequence

<220>
<223> Probe

<220>
<221> misc_feature
<222> 1
<223> N = C labelled with carboxyfluorescein

<400> 10
naccatctca gccacagc 18

<210> 11
<211> 57
<212> DNA
<213> Homo Sapiens

<400> 11
cgccagaagt gcggctggga tccggcggcc acctgcacct gcgtatctct cgggccg 57

<210> 12
<211> 89
<212> DNA
<213> Homo Sapiens

<400> 12

actatggcct tgtctgggat ctcagatttt ggcaacatct cagccacagc gggtgaaaac 60
tacagtgtcg tctcctgggt ccccaagga 89

<210> 13
<211> 137
<212> DNA
<213> Homo Sapiens

<400> 13

agcttatcat gcctggtcaa gaagcaggcc ttgggcaggt tccgctgatc gtgggcatct 60
tgctggtgtt gatggctgtg gtccttgcat tatgaagcaa gacttctccg taccccctctg 120
atatataggc gcagact 137

<210> 14
<211> 70
<212> DNA
<213> Homo Sapiens

<400> 14

tctgctggta tttttctgta aagaccattt gcaaaattgt aacctaatac aaagtgtagc 60
cttcttccaa 70

<210> 15
<211> 1220
<212> DNA
<213> Homo Sapiens

<400> 15

agtcccagct cagagccgca acctgcacag ccatgcccgg gcaagaactc aggacggtga 60
atggctctca gatgctcctg gtgttgctgg tgctctcgtg gctgccgcat gggggcgccc 120
tgtctctggc cgaggcgagc cgcgcaagtt tcccgggacc ctcagagttg cactccgaag 180
actccagatt ccgagagttg cggaaacgct acgaggacct gctaaccagg ctgcgggcca 240
accagagctg ggaagattcg aacaccgacc tcgtcccggc ccctgcagtc cggatactca 300
cgccagaagt gcggctggga tccggcggcc acctgcacct gcgtatctct cgggccgccc 360
ttcccgaggg gctccccgag gcctcccgcc ttcaccgggc tctgttccgg ctgtccccga 420
cggcgtcaag gtcgtgggac gtgacacgac cgctgcggcg tcagctcagc cttgcaagac 480
cccaggcgcc cgcgctgcac ctgcgactgt cgccgccgcc gtcgcagtcg gaccaactgc 540
tggcagaatc ttcgtccgca cggccccagc tggagttgca cttgcggccg caagccgcca 600
gggggcgccg cagagcgcgt gcgcgcaacg gggaccactg tccgctcggg cccgggcgtt 660
gctgccgtct gcacacggtc cgcgcgtcgc tggaagacct gggctgggcc gattgggtgc 720
tgtcgccacg ggaggtgcaa gtgaccatgt gcatcggcgc gtgcccgagc cagttccggg 780
cggcaaacat gcacgcgcag atcaagacga gcctgcaccg cctgaagccc gacacggtgc 840
cagcgccctg ctgcgtgccc gccagctaca atcccatggt gctcattcaa aagaccgaca 900
ccggggtgtc gctccagacc tatgatgact tgttagccaa agactgccac tgcatatgag 960
cagtcctggt ccttccactg tgcacctgcg cggaggacgc gacctcagtt gtcctgccct 1020
gtggaatggg ctcaaggttc ctgagacacc cgattcctgc ccaaacagct gtatttatat 1080
aagtctgtta tttattatta atttattggg gtgaccttct tggggactcg gggctggtc 1140
tgatggaact gtgtatttat ttaaaactct ggtgataaaa ataaagctgt ctgaactgtt 1200
aaaaaaaaaa aaaaaaaaaa 1220

<210> 16
<211> 2143
<212> DNA
<213> Homo Sapiens

<400> 16

```
agtgcctttg gttgctggag ggaagaacac aatggatctg gtgctaaaaa gatgccttct     60
tcatttggct gtgataggtg ctttgctggc tgtgggggct acaaaagtac ccagaaacca    120
ggactggctt ggtgtctcaa ggcaactcag aaccaaagcc tggaacaggc agctgtatcc    180
agagtggaca gaagcccaga gacttgactg ctggagaggt ggtcaagtgt ccctcaaggt    240
cagtaatgat gggcctacac tgattggtgc aaatgcctcc ttctctattg ccttgaactt    300
ccctggaagc caaaaggtat tgccagatgg gcaggttatc tgggtcaaca ataccatcat    360
caatgggagc caggtgtggg gaggacagcc agtgtatccc caggaaactg acgatgcctg    420
catcttccct gatggtggac cttgcccatc tggctcttgg tctcagaaga gaagctttgt    480
ttatgtctgg aagacctggg gccaatactg gcaagttcta gggggcccag tgtctgggct    540
gagcattggg acaggcaggg caatgctggg cacacacacc atggaagtga ctgtctacca    600
tcgccgggga tcccggagct atgtgcctct tgctcattcc agctcagcct tcaccattac    660
tgaccaggtg cctttctccg tgagcgtgtc ccagttgcgg gccttggatg gagggaacaa    720
gcacttcctg agaaatcagc ctctgacctt tgccctccag ctccatgacc ccagtggcta    780
tctggctgaa gctgacctct cctacacctg ggactttgga gacagtagtg gaaccctgat    840
ctctcgggca cttgtggtca ctcatactta cctggagcct ggcccagtca ctgcccaggt    900
ggtcctgcag gctgccattc ctctcacctc ctgtggctcc tccccagttc caggcaccac    960
agatgggcac aggccaactg cagaggcccc taacaccaca gctggccaag tgcctactac   1020
agaagttgtg ggtactacac ctggtcaggc gccaactgca gagccctctg gaaccacatc   1080
tgtgcaggtg ccaaccactg aagtcataag cactgcacct gtgcagatgc caactgcaga   1140
gagcacaggt atgacacctg agaaggtgcc agtttcagag gtcatgggta ccacactggc   1200
agagatgtca actccagagg ctacaggtat gacacctgca gaggtatcaa ttgtggtgct   1260
ttctggaacc acagctgcac aggtaacaac tacagagtgg gtggagacca cagctagaga   1320
gctacctatc cctgagcctg aaggtccaga tgccagctca atcatgtcta cggaaagtat   1380
tacaggttcc ctgggccccc tgctggatgg tacagccacc ttaaggctgg tgaagagaca   1440
agtcccccctg gattgtgttc tgtatcgata tggttccttt tccgtcaccc tggacattgt   1500
ccagggtatt gaaagtgccg agatcctgca ggctgtgccg tccggtgagg gggatgcatt   1560
tgagctgact gtgtcctgcc aaggcgggct gcccaaggaa gcctgcatgg agatctcatc   1620
gccagggtgc cagcccccctg cccagcggct gtgccagcct gtgctaccca gcccagcctg   1680
ccagctggtt ctgcaccaga tactgaaggg tggctcgggg acatactgcc tcaatgtgtc   1740

tctggctgat accaacagcc tggcagtggt cagcacccag cttatcatgc ctggtcaaga   1800
agcaggcctt gggcaggttc cgctgatcgt gggcatcttg ctggtgttga tggctgtggt   1860
ccttgcatct ctgatatata ggcgcagact tatgaagcaa gacttctccg taccccagtt   1920
gccacatagc agcagtcact ggctgcgtct accccgcatc ttctgctctt gtcccattgg   1980
tgagaatagc cccctcctca gtgggcagca ggtctgagta ctctcatatg atgctgtgat   2040
tttcctggag ttgacagaaa cacctatatt tcccccagtc ttccctggga gactactatt   2100
aactgaaata aatactcaga gcctgaaaaa aaaaaaaaaa aaa                      2143
```

<210> 17
<211> 4525
<212> DNA
<213> Homo Sapiens

<400> 17

```
gcgcggtgcc gccgggaaag atggtcgtgg cgctgcggta cgtgtggcct ctcctcctct   60
gcagcccctg cctgcttatc cagatccccg aggaatatga aggacaccat gtgatggagc   120
cacctgtcat cacggaacag tctccacggc gcctggttgt cttccccaca gatgacatca   180
gcctcaagtg tgaggccagt ggcaagcccg aagtgcagtt ccgctggacg agggatggtg   240
tccacttcaa acccaaggaa gagctgggtg tgaccgtgta ccagtcgccc cactctggct   300
ccttcaccat cacgggcaac aacagcaact ttgctcagag gttccagggc atctaccgct   360
gctttgccag caataagctg ggcaccgcca tgtcccatga gatccggctc atggccgagg   420
gtgcccccaa gtggccaaag gagacagtga agcccgtgga ggtggaggaa ggggagtcag   480
tggttctgcc ttgcaaccct cccccaagtg cagagcctct ccggatctac tggatgaaca   540
gcaagatctt gcacatcaag caggacgagc gggtgacgat gggccagaac ggcaacctct   600
actttgccaa tgtgctcacc tccgacaacc actcagacta catctgccac gcccacttcc   660
caggcaccag gaccatcatt cagaaggaac ccattgacct ccgggtcaag gccaccaaca   720
gcatgattga caggaagccg cgcctgctct tccccaccaa ctccagcagc cacctggtgg   780
ccttgcaggg gcagccattg gtcctggagt gcatcgccga gggctttccc acgcccacca   840
tcaaatggct gcgccccagt ggccccatgc cagccgaccg tgtcacctac cagaaccaca   900
acaagaccct gcagctgctg aaagtgggcg aggaggatga tggcgagtac cgctgcctgg   960
ccgagaactc actgggcagt gcccggcatg cgtactatgt caccgtggag gctgccccgt   1020
actggctgca caagccccag agccatctat atgggccagg agagactgcc cgcctggact   1080
gccaagtcca gggcaggccc caaccagagg tcacctggag aatcaacggg atccctgtgg   1140
aggagctggc caaagaccag aagtaccgga ttcagcgtgg cgccctgatc ctgagcaacg   1200
tgcagcccag tgacacaatg gtgacccaat gtgaggcccg caaccggcac gggctcttgc   1260
tggccaatgc ctacatctac gttgtccagc tgccagccaa gatcctgact gcggacaatc   1320
agacgtacat ggctgtccag ggcagcactg cctaccttct gtgcaaggcc ttcggagcgc   1380
ctgtgcccag tgttcagtgg ctggacgagg atgggacaac agtgcttcag gacgaacgct   1440
tcttccccta tgccaatggg accctgggca ttcgagacct ccaggccaat gacaccggac   1500
gctacttctg cctggctgcc aatgaccaaa acaatgttac catcatggct aacctgaagg   1560
ttaaagatgc aactcagatc actcaggggc cccgcagcac aatcgagaag aaaggttcca   1620
gggtgacctt cacgtgccag gcctcctttg acccctcctt gcagcccagc atcacctggc   1680
gtggggacgg tcgagacctc caggagcttg gggacagtga caagtacttc atagaggatg   1740
ggcgcctggt catccacagc ctggactaca gcgaccaggg caactacagc tgcgtggcca   1800
gtaccgaact ggatgtggtg gagagtaggg cacagctctt ggtggtgggg agccctgggc   1860
cggtgccacg gctggtgctg tccgacctgc acctgctgac gcagagccag gtgcgcgtgt   1920
cctggagtcc tgcagaagac cacaatgccc ccattgagaa atatgacatt gaatttgagg   1980
acaaggaaat ggcgcctgaa aaatggtaca gtctgggcaa ggttccaggg aaccagacct   2040
ctaccaccct caagctgtcg ccctatgtcc actacacctt tagggttact gccataaaca   2100
aatatggccc cggggagccc agcccggtct ctgagactgt ggtcacacct gaggcagccc   2160
cagagaagaa ccctgtggat gtgaaggggg aaggaaatga gaccaccaat atggtcatca   2220
cgtggaagcc gctccggtgg atggactgga acgcccccca ggttcagtac cgcgtgcagt   2280
ggcgccctca ggggacacga gggccctggc aggagcagat tgtcagcgac cccttcctgg   2340
tggtgtccaa cacgtccacc ttcgtgccct atgagatcaa agtccaggcc gtcaacagcc   2400
agggcaaggg accagagccc caggtcacta tcggctactc tggagaggac taccccccagg   2460
caatccctga gctggaaggc attgaaatcc tcaactcaag tgccgtgctg gtcaagtggc   2520
ggccggtgga cctggcccag gtcaagggcc acctccgcgg atacaatgtg acgtactgga   2580
gggagggcag tcagaggaag cacagcaaga gacatatcca caaagaccat gtggtggtgc   2640
ccgccaacac caccagtgtc atcctcagtg gcttgcggcc ctatagctcc taccacctgg   2700
aggtgcaggc ctttaacggg cgaggatcgg ggcccgccag cgagttcacc ttcagcaccc   2760
cagagggagt gcctggccac cccgaggcgt tgcacctgga gtgccagtcg aacaccagcc   2820
tgctgctgcg ctggcagccc ccactcagcc acaacggcgt gctcaccggc tacgtgctct   2880
cctaccaccc cctggatgag gggggcaagg ggcaactgtc cttcaacctt cgggaccccg   2940
aacttcggac acacaacctg accgatctca gcccccacct gcggtaccgc ttccagcttc   3000
aggccaccac caaagagggc cctggtgaag ccatcgtacg ggaaggaggc actatggcct   3060
tgtctgggat ctcagatttt ggcaacatct cagccacagc gggtgaaaac tacagtgtcg   3120
tctcctgggt ccccaaggag ggccagtgca acttcaggtt ccatatcttg ttcaaagcct   3180
tgggagaaga gaagggtggg gcttcccttt cgccacagta tgtcagctac aaccagagct   3240
```

```
cctacacgca gtgggacctg cagcctgaca ctgactacga gatccacttg tttaaggaga   3300
ggatgttccg gcaccaaatg gctgtgaaga ccaatggcac aggccgcgtg aggctccctc   3360
ctgctggctt cgccactgag ggctggttca tcggctttgt gagtgccatc atcctcctgc   3420
tcctcgtcct gctcatcctc tgcttcatca agcgcagcaa gggcggcaaa tactcagtga   3480
aggataagga ggacacccag gtggactctg aggcccgacc gatgaaagat gagaccttcg   3540
gcgagtacag gtccctggag agtgacaacg aggagaaggc ctttggcagc agccagccat   3600
cgctcaacgg ggacatcaag cccctgggca gtgacgacag cctggccgat tatgggggca   3660
gcgtggatgt tcagttcaac gaggatggtt cgttcattgg ccagtacagt ggcaagaagg   3720
agaaggaggc ggcagggggc aatgacagct caggggccac ttcccccatc aaccctgccg   3780
tggccctaga atagtggagt ccaggacagg agatgctgtg cccctggcct tgggatccag   3840
gcccctccct ctccagcagg cccatgggag gctggagttg ggcagagga gaacttgctg   3900
cctcggatcc ccttcctacc acccggtccc cactttattg ccaaaaccca gctgcacccc   3960
ttcctgggca cacgctgctc tgccccagct tgggcagatc tcccacatgc caggggcctt   4020
tgggtgctgt tttgccagcc catttgggca gagaggctgt ggtttggggg agaagaagta   4080
ggggtggccc gaaagggtct ccgaaatgct gtctttcttg ctccctgact gggggcagac   4140
atggtggggt ctcctcagga ccagggttgg caccttcccc ctcccccagc cactccccag   4200
ccagcctggc tgggactggg aacagaactc ggtgtcccca ccatctgctg tcttttcttt   4260
gccatctctg ctccaaccgg gatgggagcc gggcaaactg gccgcggggg caggggaggc   4320
catctggaga gcccagagtc cccccactcc cagcatcgca ctctggcagc accgcctctt   4380
cccgccgccc agcccacccc atggccggct ttcaggagct ccatacacac gctgccttcg   4440
gtacccacca cacaacatcc aagtggcctc cgtcactacc tggctgcggg gcgggcacac   4500
ctcctcccac tgcccactgg ccggc                                         4525
```

## Description

### BACKGROUND OF THE INVENTION

Cutaneous malignant melanoma is a serious health are problem with at least 62,000 new, invasive melanoma cases diagnosed in people expected in 2008 in the United States, among which 8,200 will die of this disease. The incidence of melanoma continues to increase fatser than that of any othetr malignancy. Also, it is one of the most common cancers in young adults and, thus, is the number two type of cancer in terams o average years of life lost.

Certain proteins have been shown to be associated with melanoma and its metastases. These proteins or their activities have been used in immunohistochemistry to identify metastases and include L1CAM (Thies et al. (2002); Fogel et al. (2003)); and S-100 (Diego et al. (2003)). High-density microarrays have been applied to simultaneously monitor expression of thousands of genes in biologoical samples. Studies have resulted in the identification of genes differentially expressed in benign and malignant lesions, as well as genes that might be of prognostic value. Luo et al. (2001); and Wang et al. (2004). Gene expression profiling of malignant melanoma was performed with a microarray containing probes that monitor the expression of 8,150 mRNA transcripts. Bittner et al. (2000). These researchers identified several genes that might be associated with aggressive tumor behavior. In recent work, the comparison of gene expression profiles of melanoma and normal melanocyte cell lines led to the identification of differentially expressed genes and pathways modulated in melanoma. Takeuchi et al. (2004). Additionally, prostate differentiation factor GDF15, the adhesion molecule L1CAM, and kinesin-like 5, osteopontin, cathepsin B, cadherin 3, and presenilin 2 were identified as promising markers for melanoma detection. (Talantov et al. (2005); Wang et al. (2007)).
Kauffman et al. (2009) discussed five markers to differentiate melanoma from nevi, or two markers to differentiate melanoma from atypical nevi. Arenberger et al. (2005) considers RT-PCR for detection of melanoma cells, using six markers. McMasters et al. (2004) reviews data from the Sunbelt melanoma trial. Rothberg et al. (2008) discloses nuclear to non-nuclear gp100 expression as a discriminator between benign and malignant melanocytic lesions. Adema et al. (1994) characterized the melanocyte lineage-specific antigen gp100*. WO-2006-002433 discloses a method of identifying a melanoma by measuring the levels of PLAB, L1CAM andNTRK3. Wagner et al. (1997) discusses the analysis of gp100 expression in primary human tissue specimens, and its implications for melanoma immuno- and gene-therapy. Alexandrescu et al. (2010) discusses melanoma-spcific marker expression in skin biopsy tissues as a tool to facilitate melanoma diagnosis.

### SUMMARY OF THE INVENTION

The invention provides a method of identifying a melanoma in a tissue sample comprising the steps of (a) measuring the expression levels in the sample of genes encoding mRNA corresponding to SILV; (b) measuring the expression level of a gene encoding tyrosinase; and (c) measuring the expression level of SILV relative to tyrosinase; wherein the expression levels of SILV relative to tyrosinase are indicative of the presence of a melanoma.

The invention also provides the use of a kit in the method of the invention, wherein the kit comprises nucleic acid amplification and detection reagents.

### SUMMARY OF THE DISCLOSURE

The present invention provides a method of identifying a melanoma in a tissue sample, comprising assaying and measuring the expression levels in the sample for genes encoding mRNA corresponding to SILV (me20m), and tyrosinase (TYR), as defined in the claims. TYR was used as a normalization control that confirms the presence of melanocytes in the tested sample. Also disclosed herein is a method of identifying a melanoma by obtaining a tissue sample; and assaying and measuring the expression levels in the sample of genes encoding mRNA corresponding to one or both of GDF15 or L1CAM and recognized by the primer/probe sets SEQ. ID NOs.: 5-7 and 8-10, respectively, where gene expression is above a pre-determined cut-off is indicative of the presence of a melanoma.

Also disclosed herein is a method of distinguishing a malignant melanocyte from a benign melanocyte by obtaining a tissue sample; and assaying and measuring the expression levels in the sample of genes encoding SILV where the gene expression levels above pre-determined cut-off is indicative of the presence of a melanoma in the sample.

Also disclosed herein is a method of distinguishing a malignant melanocyte from a benign melanocyte by obtaining a tissue sample; and assaying and measuring the expression levels in the sample for genes encoding oen or both of GDF15 and L1CAM and recognized by the primer/probe sets SEQ/ ID NO.: 5-7 and 8-10. Gene expression levels above pre-determined cut-off is indicative of the presence of a melanoma in the sample.

Also disclosed herein is a method of determining patient treatment protocol by obtaining a tissue sample from the patient; and assaying and measuring the expression levels in the sample of genes encoding SILV where the gene expression levels above pre-determined cut-off levels are indicative of the presence of a melanoma in the sample.

Also disclosed herein is a method of determining patient treatment protocol by obtaining a tissue sample from the patient; and assaying and measuring the expression levels in the sample of genes encoding one or both of GDF15 and L1CAM recognized by the primer/probe sets SEQ. ID NOS.: 5-7 and 8-10 where the gene expression levels above pre-determined cut-off levels are indicative of the presence of a melanoma in the sample.

The final Marker is SILV and is defined herein as the gene encoding any variant, allele etc. SILV is also described as MELANOCYTE PROTEIN 17; PMEL 17; PREMELANOSOMAL PROTEIN; PMEL; GP 100; ME20; SI; SIL; D12S53E and represented by Accession No. NM_006928.3. The invention further provides the use of a kit for conducting an assay to determine the presence of melanoma in a cell sample comprising: nucleic acid amplification and detection reagents.

The invention further provides primer/probe sets for amplification and detection of PCR products obtained in the inventive methods. These sets include the following:

SEQ. ID NO: 1, SILV, Forward primer, AGCTTATCATGCCTGGTCAA
SEQ. ID NO: 2, SILV, Reverse primer, GGGTACGGAGAAGTCTTGCT
SEQ. ID NO: 3, SILV, Probe, FAM-AGGTTCCGCTATCGTGGGCAT-BHQ1
SEQ. ID NO: 4, ABI AoD*, Hs00165976_ml

Also disclosed herein are primer/probe sets for amplification and detection of PCR products obtained in the disclosed methods. These sets include the following:

SEQ. ID NO: 5, GDF15, Forward primer, CGCCAGAAGTGCGGCT
SEQ. ID NO: 6, GDF15, Reverse primer, CGGCCCGAGAGATACGC
SEQ. ID NO: 7, GDF15, MGB Pobe, FAM-ATCCGGCGGCCAC
SEQ. ID NO: 8, L1CAM, Forward primer,
ACTATGGCCTTGTCTGGGATCTC
SEQ. ID NO: 9, L1CAM, Reverse primer,
AGATATGGAACCTGAAGTTGCACTG
SEQ. ID NO: 10, L1CAM, MGB Pobe, FAM-CACCATCTCAGCCACAGC

Also disclosed herein are amplicons obtained by PCR methods utilized in the inventive methods. These amplicons include the following:

SEQ. ID NO: 11, GDF15 PCR amplicon:

CGCCAGAAGTGCGGCTGGGATCCGGCGGCCACCTGCACCTGCGTATC
TCTCGGGCCG

SEQ. ID NO: 12 L1CAM PCR amplicon:

ACTATGGCCTTGTCTGGGATCTCAGATTTTGGCAACATCTCAGCCACA
GCGGGTGAAAACTACAGTGTCGTCTCCTGGGTCCCCAAGGA

SEQ. ID NO: 13 SILV PCR amplicon:

AGCTTATCATGCCTGGTCAAGAAGCAGGCCTTGGGCAGGTTCCGCTGA
TCGTGGGCATCTTGCTGGTGTTGATGGCTGTGGTCCTTGCATTATGAA
GCAAGACTTCTCCGTACCCCTCTGATATATAGGCGCAGACT

SEQ. ID NO: 14 TYR PCR amplicon:

TCTGCTGGTATTTTTCTGTAAAGACCATTTGCAAAATTGTAACCTAAT
ACAAAGTGTAGCCTTCTTCCAA

BRIEF DESCRIPTION OF THE DRAWINGS

Figure 1 is a graph showing the performance of SILV, GDF15 and L1CAM in distibnguishing benign and malignant skin lesions.

Figure 2 is a graph plotting sensitivity versus specificity of SILV, GDF15 and L1CAM in distinguishing unequivocal melanomas and bening nevi.

Figure 3 is a graph of SILV performance in distinguishing between melanoma and atypical nevi.

Figure 4 is a graph plotting sensitivity versus specificity of SILV in distinguishing unequivocal melanomas and bening nevi.

DETAILED DESCRIPTION

The present invention provides methods of qualitatively and quantitatively identifying a melanoma, as defined in the claims. Also disclosed herein are methods of distinguishing a malignant melanocyte from a benign melanocyte; diagnosing melanocytic lesions with uncertain pathological features; and determining a melanoma patient treatment protocol. The methods further provide aids in patient prognosis, patient monitoring and drug development. The methods rely on assaying and measuring expression levels of SILV (me20m) as a final Marker for melanoma biopsy assays where a certain level of gene expression, relative to TYR normalization control, is indicative of the presence of a malignant melanocyte in the sample assayed.

The present invention focuses on the utility of identified gene expression markers to diagnose malignant melanoma among various skin lesions, including lesions with uncertain morphological features or suspicious primary melanocytic lesions termed equivocal cases using paraffin-embedded ("FFPE") tissues. This setting is where discrepancies between the opinions of dermatopathologists occur. Thus, the utility of this invention is to identify gene expression markers that differentiate benign melanocytic skin lesions from primary melanomas in gene expression assays, based on RT-PCR, for the diagnosis of melanoma in suspicious lesions.

Total RNA was isolated from 47 primary melanoma, 48 benign skin nevi, and 98 atypical/suspicious nevi including 48 atypical nevi and 50 melanomas (as assigned by dermatopathologists) tissue specimens were analyzed using RT-PCR. Differential gene expression of three melanoma specific genes, SILV, GDF15, and L1CAM, were tested by a one-step quantitative RT-PCR assay on melanoma, benign nevi and atypical/suspcious skin samples. The results demonstrated the ability of using SILV as a final Marker to differentiate clinically relevant tissue samples containing benign or malignant melanocytes.

High-density cDNA and oligonucleotide microarrays allow simultaneous monitoring of the expression of thousands of genes. Microarray technology provides a quantitative measurement of mRNA abundance and has gained acceptance as a tool for marker discovery based on gene expression. In the context of cancer research, microarray analysis has identified genes differentially expressed in benign and malignant lesions for different cancer types or that have prognostic significance. Luo et al. (2001); Su et al. (2001); Henshall et al. (2003); and Wang et al. (2004). The first gene expression profiling of malignant melanoma used a microarray containing probes for 8,150 mDNA trabscripts and identified genes that might be associated with aggressive tumor behavior. Bittner et al. (2000). Since the samples analyzed in their study did not include tissues containing normal or benign melanocytes, differentially expressed genes in malignant melanoma were not identified. In contrast to normal skin, melanocyte content in benign nevi is close to that in melanoma.

In another study, two pooled samples derived from either melanoma or benign nevi tissues were hybridized to a cDNA array and genes preferentially expressed in melanoma- or nevi-derived samples were found. Seykora et al. (2003). Other researchers used subtractive hybridization or analysis of SAGE libraries generated on melanoma cell lines, for monitoring gene expression in melanoma. Hipfel et al. (2000); and Weeraratna (2004). Recently, the comparison of gene expression profiles of a few melanoma and melanocyte cell lines led to the identification of differentially expressed genes and pathways modulated in melanoma. Hoek et al. (2004). While these studies provide a solid foundation for melanoma genomics, there is no marker that can clearly differentiate melanoma from benign tissue. Several markers currently used such as tyrosinase, HMB-45, mart-a/Melanin-a and MITF have not proiven to have prognostic value for melanocytic tumor identification Phsie etal. (2008) Consequently, these markers have found limited clincal use.

As disclosed in United States Patent Publication No. 20070154889, PCR was demonstrated to have sufficient specificity and sensitivity to detect metastasis of melanoma. In the present invention, a method with improved diagnostic performance in differentiating melanoma from non-melanoma lesions from primary skin biopsies is provided. The assay of the invention is useful in diagnosing clear-cut, or unequivocal, from suspicious, or equivocal, lesions. Thus, the instant invention may find particular utility in testing of early stage tissue samples. Preferably, a probability measurement will distinguish tissues having melanoma relative to benign melanocyte or normal tissue.

The methods of the invention employ a rapid technique for extracting nucleic acids from FFPE tissue samples and a method of amplifying and detecting nucleic acid fragments indicative of melanoma in primary skin lesions. The nucleic acid fragments qualitatively and quantitatively measure mRNA encoded by the Marker gene. Tissue samples include skin lesions derived from punch, needle, excisional or shave biopsies. The methods provided herein allow for melanoma detection in primary skin biopsy samples allowing a physician to determine whether to immediately implement an appropriate treatment protocol for early stage disease. In the methods of the invention, it is important to adequately sample the tissue used to conduct the assay. This includes proper excision and processing of the tissue sample as well as extraction of RNA. Once obtained, it is important to process the tissue samples properly so that any cancerous cells present are detected.

In one method of the invention, RNA is isolated from an FFPE tissue sample block. Any suitable commercially available paraffin kit may be used, such as High Pure RNA Paraffin Kit from Roche (Cat. #3270289). Preferably, the FFPE samples are sectioned according to the sixe of the embedded tumor as follows: ≤ 2 to 5 mm sectioned to 9 x 10 μm; ≥ 6 to 8 mm sectioned to 6 x 10 μm. Sections are de-paraffinized according to the manufacturer's instructions and the isolated RNA may be stored in RNase-free water at -80° C.

In the case of measuring mRNA levels to determine gene expression, assays can be by any means known in the art and include methods such as PCR, Rolling Circle Amplification (RCA), Ligase Chain Reaction (LCR), Strand Displacement Amplification (SDA), Nucleic Acid Sequence Based Amplification (NASBA), and others. The rapid molecular diagnostics involved are most preferably quantitative PCR methods, including QRT-PCR. Detection can be by any method known in the art including microarrays, gene chips and fluorescence.

A typical PCR includes multiple amplification steps, or cycles that selectively amplify target nucleic acid species. A typical PCR includes three steps: a denaturing step in which a target nucleic acid is denatured; an annealing step in which a set of PCR primers (forward and backward primers) anneal to complementary DNA strands; and an elongation step in which a thermostable DNA polymerase elongates the primers. By repeating this step multiple times, a DNA fragment is amplified to produce an amplicon, corresponding to the target DNA sequence. Typical PCR includes 20 or more cycles of denaturation, annealing and elongation. Often, the annealing and elongation steps can be performed concurrently, in which case the cycle contains only two steps.

In one embodiment of the invention, the RT-PCR amplification reaction may be conducted in a time so that the lengths of each of these steps can be in the seconds range, rather than minutes. Specifically, with certain new thermal cyclers being capable of generating a thermal ramp rate of at least about 5C° per second, RT-PCR amplifications in 30 minutes or less are used. More preferably, amplifications are conducted in less than 25 minutes. With this in mind, the following times provided for each step of the PCR cycle do not include ramp times. The denaturation step may be conducted for times of 10 seconds or less. In fact, some thermal cyclers have settings for "0 seconds" which may be the optimal duration of the denaturation step. That is, it is enough that the thermal cycler reaches the denaturation temperature. The annealing and elongation steps are most preferably less than 10 seconds each, and when conducted at the same temperature, the combination annealing/elongation step may be less than 10 seconds. Some homogeneous probe detection methods, may require a separate step for elongation to maximize rapid assay performance. In order to minimize both the total amplification time and the formation of non-specific side reactions, annealing temperatures are typically above 50°C. More preferably annealing temperatures are above 55°C.

A single combined reaction for RT-PCR, with no experimenter intervention, is desirable for several reasons: (1) decreased risk of experimenter error; (2) decreased risk of target or product contamination; and (3) increased assay speed. The reaction can consist of either one or two polymerases. In the case of two polymerases, one of these enzymes is typically an RNA-based DNA polymerase (reverse transcriptase) and one is a thermostable DNA-based DNA polymerase. To maximize assay performance, it is preferable to employ a form of "hot start" technology for both of these enzymatic functions. US Patents 5,411,876 and 5,985,619 provide examples of different "hot start" approaches. Preferred methods include the use of one or more thermoactivation methods that sequester one or more of the components required for efficient DNA polymerization. US Patents 5,550,044 and 5,413,924 describe methods for preparing reagents for use in such methods. US Patent 6,403,341 describes a sequestering approach that involves chemical alteration of one of the PCR reagent components. In the most preferred embodiment, both RNA- and DNA-dependent polymerase activities reside in a single enzyme. Other components that are required for efficient amplification include nucleoside triphosphates, divalent salts and buffer components. In some instances, non-specific nucleic acid and enzyme stabilizers may be beneficial.

In the preferred RT-PCR, the amounts of certain reverse transcriptase and the PCR components are atypical in order to take advantage of the faster ramp times of some thermal cyclers. Specifically, the primer concentrations are very high.

Typical gene-specific primer concentrations for reverse transcriptase reactions are less than about 20 nM. To achieve a rapid reverse transcriptase reaction on the order of one to two minutes, the reverse transcriptase primer concentration is raised to greater than 20 nM, preferably at least about 50 nM, and typically about 100 nM. Standard PCR primer concentrations range from 100 nM to 300 nM. Higher concentrations may be used in standard PCR to compensate for Tm variations. However, for the purposes herein, the referenced primer concentrations are for circumstances where no Tm compensation is needed. Proportionately higher concentrations of primers may be empirically determined and used if Tm compensation is necessary or desired. To achieve rapid PCR, the PCR primer concentrations typically are greater than 250 nM, preferably greater than about 300 nM and typically about 500 nM.

Commercially used diagnostics also preferably employ one or more internal positive control that confirms the operation of a particular amplification reaction in case of a negative result. Potential causes of false negative results that must be controlled in an RT-PCR include: inadequate RNA quantity, degradation of RNA, inhibition of RT and/or PCR and experimenter error.

In the case of measuring protein levels to determine gene expression, any method known in the art is suitable provided it results in adequate specificity and sensitivity. For example, protein levels can be measured by binding to an antibody or antibody fragment specific for the protein and measuring the amount of antibody-bound protein. Antibodies can be labeled by radioactive, fluorescent or other detectable reagents to facilitate detection. Methods of detection include, without limitation, enzyme-linked immunosorbent assay (ELISA) and immunoblot techniques.

The invention provides specificity and sensitivity sufficient to identify a malignant melanocyte in a tissue sample. The methods determine expression of a particular Marker gene, SILV, by measuring mRNA encoded by the Marker. The results presented herein show that SILV is a leading marker demonstrating clear discrimination between melanoma and benign, unequivocal cases as well as between different atypia subgroups in suspicious tissue group samples. The Marker SILV is defined herein as the gene encoding any variant, allele etc. including SEQ ID NO: 1-3.

In the methods of the invention, tyrosinase is used as a control gene to demonstrate the presence of melanocytes in the tissue sample and to normalize for melanocytic content. Tyrosinase is described by Mandelcorn-Monson et al. (2003); and US Patent No. 6,153,388 and is represented by Accession No. NM_000372. Tyrosinase is also defined as the gene encoding mRNA recognized by the ABI assay on demand (Hs00165976_m1) with PCR amplicon SEQ ID NO: 14.

The specificity of any given amplification-based molecular diagnostic relies heavily, but not exclusively, on the identity of the primer sets. The primer sets are pairs of forward and reverse oligonucleotide primers that anneal to a target DNA sequence to permit amplification of the target sequence, thereby producing a target sequence-specific amplicon. The primers must be capable of amplifying Markers of the disease state of interest. In the case of the instant invention, the Marker is directed to melanoma.

The reaction must also contain some means of detection of a specific signal. This is preferably accomplished through the use of a reagent that detects a region of DNA sequence derived from polymerization of the target sequence of interest. Preferred reagents for detection give a measurable signal differential when bound to a specific nucleic acid sequence of interest. Often, these methods involve nucleic acid probes that give increased fluorescence when bound to the sequence of interest. Typically, the progress of the reactions of the inventive methods are monitored by analyzing the relative rates of amplicon production for each PCR primer set.

The invention further includes the use of primer/probe sets in the claimed methods, wherein the sequences IDs are: SEQ, ID NOs.: 1-3. Monitoring amplicon production may be achieved by a number of detection reagents and methods, including without limitation, fluorescent primers, and fluorogenic probes and fluorescent dyes that bind double-stranded DNA. Molecular beacons, Scorpions, and other detection schemes may also be used. A common method of monitoring a PCR employs a fluorescent hydrolysis probe assay. This method exploits the 5' nuclease activity of certain thermostable DNA polymerases (such as Taq or Tfl DNA polymerases) to cleave an oligomeric probe during the PCR process.

Disclosed herein are amplicons obtained by PCR methods utilized in the inventive methods. These amplicons include the sequences: SEQ. ID Nos: 11-14.

The oligomer is selected to anneal to the amplified target sequence under elongation conditions. The probe typically has a fluorescent reporter on its 5' end and a fluorescent quencher of the reporter at the 3' end. So long as the oligomer is intact, the fluorescent signal from the reporter is quenched. However, when the oligomer is digested during the elongation process, the fluorescent reporter is no longer in proximity to the quencher. The relative accumulation of free fluorescent reporter for a given amplicon may be compared to the accumulation of the same amplicons for a control sample and/or to that of a control gene, such as, without limitation, β-Actin or PBGD to determine the relative abundance of a given cDNA product of a given RNA in a RNA population. Products and reagents for the fluorescent hydrolysis probe assay are readily available commercially, for instance from Applied Biosystems.

The most preferred detection reagents are TaqMan® probes (Roche Diagnostics, Branchburg, NJ) and they are described in US Patents 5,210,015; 5,487,972; and 5,804,375. Essentially, these probes involve nucleic acid detection by virtue of the separation of a fluor-quencher combination on a probe through the 5'-3' exonuclease activity of the polymerase used in the PCR. Any suitable fluorophore can be used for any of the Markers or controls. Such fluorophores include, without limitation, Texas Red, Cal Red, Fam, Cy3 and Cy5. In one embodiment, the following fluorophores correspond to the noted Markers: PLAB: Fam; L1CAM: Texas Red or Cal Red, tyrosinase: C1; PBGD: Cy5. Equipment and software also are readily available for controlling and monitoring amplicon accumulation in PCR and QRT-PCR including the Smart Cycler thermocylcer commercially available from Cepheid of Sunnyvale, California, and the ABI Prism 7900 Sequence Detection System, commercially available from Applied Biosystems.

In the commercialization of the described methods for QRT-PCR certain kits for detection of specific nucleic acids are particularly useful. In one embodiment, the kit includes reagents for amplifying and detecting Markers. Optionally, the kit includes sample preparation reagents and or articles (e.g., tubes) to extract nucleic acids from lymph node tissue. The kits may also include articles to minimize the risk of sample contamination (e.g., disposable scalpel and surface for lymph node dissection and preparation).

In a preferred kit, reagents necessary for the one-tube QRT-PCR process described above are included such as reverse transcriptase, a reverse transcriptase primer, a corresponding PCR primer set (preferably for Markers and controls), a thermostable DNA polymerase, such as Taq polymerase, and a suitable detection reagent(s), such as, without limitation, a scorpion probe, a probe for a fluorescent hydrolysis probe assay, a molecular beacon probe, a single dye primer or a fluorescent dye specific to double-stranded DNA, such as ethidium bromide. The primers are preferably in quantities that yield the high concentrations described above. Thermostable DNA polymerases are commonly and commercially available from a variety of manufacturers. Additional materials in the kit may include: suitable reaction tubes or vials, a barrier composition, typically a wax bead, optionally including magnesium; reaction mixtures (typically 10X) for the reverse transcriptase and the PCR stages, including necessary buffers and reagents such as dNTPs; nuclease-or RNase-free water; RNase inhibitor; control nucleic acid(s) and/or any additional buffers, compounds, co-factors, ionic constituents, proteins and enzymes, polymers, and the like that may be used in reverse transcriptase and/or PCR stages of QRT-PCR. Optionally, the kits include nucleic acid extraction reagents and materials. Instructions are also preferably included in the kits.

**Example 1**

**Patient Clinical and Pathological Characteristics**

204 FFPE skin biopsy tissue specimens were selected from patients with primary melanocytic skin lesions diagnosed at Georgetown University Hospital. The patient series included specimens with 102 unequivocal features of invasive melanoma or benign nevi and 102 specimens with various degrees of cellular atypia. These atypical specimens were initially classified as suspicious/atypical and subsequently resolved by expert dermatopathologists as atypical nevi or malignant melanoma. Two patient samples were excluded because of insufficient RNA yield (less than 350 ng) after a sample preparation step. An additional nine RNA samples were excluded due to the failure of the PCR control. The final sample set eligible for analysis consisted of 193 biopsy tissues (95% of the original sample set) representing 47 melanomas, 48 benign nevi and 98 atypical/suspicious, including 48 atypical nevi and 50 melanomas as assigned by dermatopathologists. A summary of the pathological and clinical characteristics of the melanoma samples is shown in Table 1.

**Table 1.**

| Patient Characteristics | Advanced Melanoma | Melanoma | Severe Atypia | Moderate Atypia | Benign Nevi |
|---|---|---|---|---|---|
| **Mean Age** | 59 | 51 | 42 | 440 | 42 |
| | | | | | |
| **Gender** | | | | | |
| Female | 8 | 35 | 20 | 8 | 33 |
| Male | 6 | 48 | 15 | 5 | 15 |
| | | | | | |
| **T Stage (thickness)** | | | | | |
| Tis | | 21 | | | |
| T1 (< 1 mm) | 1 | 62 | | | |
| T2 (1.01 - 2 mm) | 7 | | | | |
| T3 (2.01 - 4 mm) | 3 | | | | |
| T4 (> 4 mm) | 1 | | | | |
| M | 2 | | | | |

(continued)

| Patient Characteristics | Advanced Melanoma | Melanoma | Severe Atypia | Moderate Atypia | Benign Nevi |
|---|---|---|---|---|---|
| | | | | | |
| **Diagnosis** | | | | | |
| Superficial spreading melanoma | 6 | 50 | | | |
| Nodular melanoma | 5 | | | | |
| Melanoma in-situ | | 21 | | | |
| Lentigo maligna | | 11 | | | |
| Melanoma other | 3 | 1 | | | |
| Compound nevus | | | | | 31 |
| Inflamed compound nevus | | | | | 13 |
| Intradermal nevus | | | | | 4 |
| Atypical nevus severe atypia | | | 2 | | |
| Compound nevus severe atypia | | | 29 | | |
| Compound nevus moderate atypia | | | | 13 | |
| Junctional nevus severe atypia | | | 4 | | |
| **Total n per category** | 14 | 83 | 35 | 13 | 48 |
| Unequivocal melanoma, n=47 | 12 | 35 | | | |
| Atypical melanoma, n=50 | 2 | 48 | | | |
| Unequivocal benign, n=48 | | | | | 48 |
| Atypical nevi, n=48 | | | 35 | 13 | |

Samples were ordered from most benign to most malignant cases, based on the provided clinical data by pathology. Using a histological diagnosis, five major categories were created: advanced melanoma, melanoma, severe atypia, moderate atypia and benign nevi, with each of the 193 samples fitting into one of these groups. Lentigo maligna melanoma, melanoma *in situ,* and superficial invasive melanomas were combined into a single melanoma category. Two sets of melanomas with advanced features (superficial spreading with T2 and greater and nodular or metastatic) were added into an advanced melanoma group. A binary classification was based on splitting advanced melanomas and melanomas into malignant, and the remaining classes as benign. This stratification contained 97 malignant cases with 47 unequivocal and 50 severely atypical lesions classified as melanomas, and 96 benign cases representing 48 benign and 48 atypical nevi. All further data analysis described is presented for unequivocal cases classified into one of the 3 groups: advanced melanoma, melanoma and benign and for equivocal cases classified into one of the 4 groups: advanced melanoma, melanoma, severe and moderate atypia.

**Example 2**

**Tissue Preparation**

Two hundred four tissue samples were collected from individuals diagnosed with primary melanocytic skin lesions. All samples were collected using excisional, punch, or shave biopsy depending on lesion size, depth, and physician judgment and embedded in FFPE blocks.

Total RNA was isolated from FFPE blocks using a standard High Pure RNA paraffin Kit from Roche (catalogue # 3270289) with the following modifications. Paraffin embedded tissue sampes were sectioned according to the size of the embedded tumor (2-5 mmm or smaller = 9 x 10 $\mu$m, 6-8 mm or greater = 6 x 10 $\mu$m). Sections were de-paraffinized according to the manufacturer's instructions. The isolated RNA was stored in RNase free water at -80° C until used.

The distribution by biopsy type and extracted RNA yield are presented in Tables 2a and 2b, respectively. Median RNA yields, corresponding to a total average of 10.5, 4.5 and 6 slides were equivalent among all three types of biopsies. No bias in assay perofamnce was observed based on the differences in biopsy techniques.

Table 2a

| Pathology Diagnosis | Excision | Punch | Shave | Total |
|---|---|---|---|---|
| Melanoma | 21 | 7 | 23 | 51 |
| Benign | 6 | 4 | 41 | 51 |
| Atypical/Suspicious | 39 | 22 | 41 | 102 |
| Total | 66 (32%) | 33 (16%) | 105 (52%) | 204 (100%) |

Table 2b

| Biopsy Type | Number (%) | Median Size | Section # | Median RNA Yield (ng) | Range (ng) |
|---|---|---|---|---|---|
| Excision | 66 (32) | 13x10.5x8 | 3-6 | 1360.6 | 496-26879 |
| Punch | 33 (16) | 8x7x3 | 6 | 1534.1 | 397-7368 |
| Shave | 105 (52) | 7x6x1.5 | 9-12 | 1400.4 | 318-12140 |

**Example 3**

**Single One-step qRTPCR Assays Using RNA-specific Primers and Cutoff Establishment**

Evaluation of expression of selected genes was carried out with one-step RT-PCR with RNA from melanoma, benign nevi, and atypical/suspicious FFPE tissue. The specifmens included two seriesof samples: 1) unequivocal, or clear-cut, menaoma and benign nevi cases and 2.) samples with various degrees of atypia. Tyrosinase ("TYR") was used as a housekeeping gene to control for the input quantity and quality of RNA in the reactions. DNase treatment was not used. Instead, primers or probes were designed to span an intron so they would not report on genomic DNA. All primer/probe sets were pre-screened on a set of 20 total RNA specimens isolated from 10 melanoma and 10 benign nevi FFPE tissues from a commercial vendor (Oncomatrix). The best performing primer-probe set was selected for each of the four markers. The sequences are listed in Table 3 below.

The gene expression markers GDF-15, SILV, and L1CAM along with the normalization control tyrosinase ("TYR") were tested in the melanoma biopsy assay using a single reaction RT-PCR format on the ABI7900 platform. Single, one step qRT-PCR assays were run in accordance with the following protocol. 50 ng of total RNA was used for qRT-PCR. The total RNA was reverse transcribed using 40X Multiscribe and RNase inhibitor mix contained in the TAQMAN® One Step PCR Master Mix Reagents Kit (Applied Biosystems, Foster City, CA). The cDNA was then subjected to the 2x Master Mix using UNG and PCR amplification was carried out on the ABI 7900 HT Sequence Detection System (Applied Biosystems, Foster City, CA) in the 384-well format using a 10μl reaction size. Each reaction was composed of 5.0 μl of 2X One Step RT-PCR Master Mix, 0.5 μl of primer/probe mix, 0.25 μl of 40X Multiscribe enxyme, and RNase Inihibitor Mix, 0.25 μl of dNTP and 4 μl of 12.5 ng/μl total RNA. The final primer/probe mix was composed of a final concentration of 900 nM of forward and reverse primers, listed in Table 3, and 250 nM of fluorescent probe. The dNTP mix contained a final concentration of 20 mM each of dATP, dGTP, dCTP, and dTTP. The reaction mixture was incubated at 48° C for 30 min. for the reverse transcription, followed by an Amplitaq activation step of 95° C for 10 minutes, and finally 40 cycles of 95° C for 15 sec. denaturing and 60° C for 1 minute anneal and extension. Sequences used in the reactions were as follows, each written in the 5' to 3' direction.

Table 3

| Symbol | Primer/Probe | Sequence | ID. No. |
|---|---|---|---|
| GDF15 | Forward | CGCCAGAAGTGCGGCT | 5 |
| | Reverse | CGGCCCGAGGATACGC | 6 |
| | MGB Probe | FAM-ATCCGGCGGCCAC | 7 |

(continued)

| Symbol | Primer/Probe | Sequence | ID. No. |
|---|---|---|---|
| L1CAM | Forward | ACTATGGCCTTGTCTGGGATCTC | 8 |
| | Reverse | AGATATGGAACCTGAAGTTGCACTG | 9 |
| | MGB probe | FAM-CACCATCTCAGCCACAGC | 10 |
| SILV | Forward | AGCTTATCATGCCTGGTCAA | 1 |
| | Reverse | GGGTACGGAGAAGTCTTGCT | 2 |
| | Probe | FAM-AGGTTCCGCTATCGTGGGCAT-BHQ1 | 3 |
| TYR | ABI AoD* | Hs00165976_m1 | 4 |

For each sample ΔCt=Ct (Target Gene) - Ct TYR was calculated. ΔCt has been widely used in clinical RT-PCR assays and was chosen as a straightforward method. Cronin et al. (2004).

The Ct values obtained from ABI7900 output files were used for data analysis. In the single reaction assay configuration, only samples genmerating TYR Ct < 30 were analyzed, The Ct values for each of the markers are presented as raw Cts normalized against the melanocyte-specific marker, TYR, using the following equation:

$$Ct(normalized) = Ct(marker) - CT(TYR)$$

Diagnosis rendered by assay was compared with dermatopathological examination. To estimate assay performance, AUC values were calculated based on ROC curve analysis using R software package version 2.5.0. (team RDC www.r-project.org).

For clear-cut (unequivocal) cases, increased expression was demonstrated in melanoma compared to benign lesions for the three melanoma-specific markers (Table 4, Figure 1a). Significant differential expression was observed for SILV and GDF15 between benign nevi and melanoma samples in clear-cut (unequivocal) cases (2.8- and 1.2-fold with p-values <0.001 and 0.003, respectively). However, L1CAM demonstrated much less differentiation with a fold change of 0.2 and no statistical significance for the difference (p=0.47) between benign and malignant clear-cut cases (Figure 1). Thus, this marker was excluded from further analysis. SILV demonstrated the best performance with a linear response across the three patient groups (advanced melanoma, melanoma, and benign cases), representing continuously changing degrees of disease status as defined by pathology.

Table 4

| Marker Normalized to TYR | Advanced Melanoma | Melanoma | Benign Nevi | P-values (benign v malignant) | AUC (classification as benign or malignant) |
|---|---|---|---|---|---|
| L1CAM | 6.85 | 7.5 | 7.66 | 0.47 | 0.49 |
| SILV | 1.18 | 2.09 | 4.93 | <0.001 | 0.94 |
| GDF15 | 5.02 | 7.17 | 8.34 | 0.003 | 0.67 |

The performance of SILV and GDF15 was assessed for differentiation between unequivocal melanomas and benign nevi using a univariate ROC curve analysis. As shown in Figure 2, AUC values were 0.94 and 0.67, respectively. Based on multivariate analysis with a linear regression model, the combination of SILV and GDF15 did not improve assay performance beyond the AUV of 0.94 in unequivocal cases. Therefore, GDF15 was not pursued further for analysis of suspicious (equivocal cases). Finally, normliazation to TYR improved performance of SILV to 0.94 compared to 0.78 when using raw Ct values.

The performance of SILV was assessed further by compring suspicious cases to unequivocal bening cases. The average ΔCt of SILV in the equivocal samples in each by histology, excluding advanced melanoma since n=2, was compared to the average ΔCt of the unequivocal bening group. The average ΔCt values and p-values for t-test comparisions to the unequivocal begin samples are listed in Table 5 below. SILV was significantly different between suspicious melanoma and each suspicious atypical group: melanoma versus severe atypia with a p-value =0.0077 and melanoma vs. moderate atypia with a p-value = 0.0009.

Figures 1 through 4 confirm that SILV is the leading marker and demonstrated clear discrimination between melanoma and benign equivocal cases as well as between different atypia subgroups in the suspicious group of tissue samples.

**Table 5**

| **Marker Normalized to TYR** | Normalized ΔCt Values | | | | P-Values | | |
|---|---|---|---|---|---|---|---|
| | Melanoma | Severe Atypia | Moderate Atypia | Benign Unequivocal | Benign v. Moderate | Benign v. Severe | Benign v. Melanoma |
| SILV | 1.7 | 2.49 | 3.15 | 4.93 | 0.002 | 3.35E-09 | 9.98E-16 |

From a dermatopathologist perspective, there is no single criterion to determine whether a pigmented lesion is a severely atypical nevus or has reached the threshold for melanoma. Dermatopathologists wrestle with at least 10 separate histologic features. No one immunohistochemical marker is able to distinguish benign from malignant melanocytic proliferations either.

The invention herein presents a melanoma biopsy assay with improved diagnostic performance in differentiating melanoma from melanocytic lesions by identifying and validating a specific genetic signature of melanoma. The testing results demonstrate a progressive increase in at least two genes that are differentially expressed in melanoma: SILV and GDF15. However, based on multivariate analysis with a linear regression model, addition of GDF15 did not improve SILV performance beyond the AUC of 0.94 in the clear-cut cases. Therefore, SILV is designated as the final marker for the melanoma biopsy assay. A significant difference was also observed between severely atypical nevi and melanoma for SILV with a p-value of 0.0077 making this marker applicable for diagnosis of both clear-cut (unequivocal) and suspicious (equivocal) cases, the latter being the most difficult challenge for expert pathologists.

Table 6.

| Sequence Descriptions, Names and SEQ ID NOs | | | | | |
|---|---|---|---|---|---|
| SEQ ID No. | Affymetrix PSID | Gene symbol Sequence name, | Accession No. | 5' - 3' Sequence | Gene name in NCBI |
| 1 | 209848_s_at | SILV Forward primer | NM_006928.3 | AGCTTATCATGCCTGGTCAA | Homo sapiens silver homolog (mouse) (SILV), mRNA |
| 2 | | SILV Reverse primer | | GGGTACGGAGAAGTCTTGCT | |
| 3 | | SILV Probe | | FAM-AGGTTCCGCTATCGTGGGCAT-BHQ1 | |
| 4 | 206630_at | TYR ABI AoD*, | NM_000372.4 | Hs00165976_m1 | Homo sapiens tyrosinase (oculocutaneous albinism IA) (TYR), mRNA |
| 5 | 221577_x_at | GDF15 Forward primer | NM_004864.1 | CGCCAGAAGTGCGGCT | Homo sapiens growth differentiation factor 15 (GDF15), mRNA |
| 6 | | GDF15 Reverse primer | | CGGCCCGAGAGATACGC | |
| 7 | | GDF15 MGB Probe | | FAM-ATCCGGCGGCCAC | |

(continued)

| Sequence Descriptions, Names and SEQ ID NOs | | | | | |
|---|---|---|---|---|---|
| SEQ ID No. | Affymetrix PSID | Gene symbol Sequence name, | Accession No. | 5' - 3' Sequence | Gene name in NCBI |
| 8 | 204585_s_at | L1CAM Forward primer | NM_000425.2 | ACTATGGCCTTGTCTGGGA TCTC | Homo sapiens L1 cell adhesion molecule, mRNA |
| 9 | | L1CAM Reverse primer | | AGATATGGAACCTGAAGTT GCACTG | |
| 10 | | L1CAM MGB Probe | | FAM- CACCATCTCAGCCACAGC | |

**Full-legth** sequences of 4 markers as provided in NCBI **database.**

>gi|113722118|ref|NM_000372.4| Homo sapiens tyrosinase (oculocutaneous albinism IA) (TYR), mRNA

ATCACTGTAGTAGTAGCTGGAAAGAGAAATCTGTGACTCCAATTAGCCAGTTCCTGCAGACCTTGTGAGG
ACTAGAGGAAGAATGCTCCTGGCTGTTTTGTACTGCCTGCTGTGGAGTTTCCAGACCTCCGCTGGCCATT
TCCCTAGAGCCTGTGTCTCCTCTAAGAACCTGATGGAGAAGGAATGCTGTCCACCGTGGAGCGGGGACAG
GAGTCCCTGTGGCCAGCTTTCAGGCAGAGGTTCCTGTCAGAATATCCTTCTGTCCAATGCACCACTTGGG
CCTCAATTTCCCTTCACAGGGGTGGATGACCGGGAGTCGTGGCCTTCCGTCTTTTATAATAGGACCTGCC
AGTGCTCTGGCAACTTCATGGGATTCAACTGTGGAAACTGCAAGTTTGGCTTTTGGGGACCAAACTGCAC
AGAGAGACGACTCTTGGTGAGAAGAAACATCTTCGATTTGAGTGCCCCAGAGAAGGACAAATTTTTTGCC
TACCTCACTTTAGCAAAGCATACCATCAGCTCAGACTATGTCATCCCCATAGGGACCTATGGCCAAATGA
AAAATGGATCAACACCCATGTTTAACGACATCAATATTTATGACCTCTTTGTCTGGATGCATTATTATGT
GTCAATGGATGCACTGCTTGGGGGATCTGAAATCTGGAGAGACATTGATTTTGCCCATGAAGCACCAGCT
TTTCTGCCTTGGCATAGACTCTTCTTGTTGCGGTGGGAACAAGAAATCCAGAAGCTGACAGGAGATGAAA
ACTTCACTATTCCATATTGGGACTGGCGGGATGCAGAAAAGTGTGACATTTGCACAGATGAGTACATGGG
AGGTCAGCACCCCACAAATCCTAACTTACTCAGCCCAGCATCATTCTTCTCCTCTTGGCAGATTGTCTGT
AGCCGATTGGAGGAGTACAACAGCCATCAGTCTTTATGCAATGGAACGCCCGAGGGACCTTTACGGCGTA
ATCCTGGAAACCATGACAAATCCAGAACCCCAAGGCTCCCCTCTTCAGCTGATGTAGAATTTTGCCTGAG
TTTGACCCAATATGAATCTGGTTCCATGGATAAAGCTGCCAATTTCAGCTTTAGAAATACACTGGAAGGA
TTTGCTAGTCCACTTACTGGGATAGCGGATGCCTCTCAAAGCAGCATGCACAATGCCTTGCACATCTATA
TGAATGGAACAATGTCCCAGGTACAGGGATCTGCCAACGATCCTATCTTCCTTCTTCACCATGCATTTGT
TGACAGTATTTTTGAGCAGTGGCTCCGAAGGCACCGTCCTCTTCAAGAAGTTTATCCAGAAGCCAATGCA
CCCATTGGACATAACCGGGAATCCTACATGGTTCCTTTTATACCACTGTACAGAAATGGTGATTTCTTTA
TTTCATCCAAAGATCTGGGCTATGACTATAGCTATCTACAAGATTCAGACCCAGACTCTTTTCAAGACTA
CATTAAGTCCTATTTGGAACAAGCGAGTCGGATCTGGTCATGGCTCCTTGGGGCGGCGATGGTAGGGGCC
GTCCTCACTGCCCTGCTGGCAGGGCTTGTGAGCTTGCTGTGTCGTCACAAGAGAAAGCAGCTTCCTGAAG
AAAAGCAGCCACTCCTCATGGAGAAAGAGGATTACCACAGCTTGTATCAGAGCCATTTATAAAAGGCTTA
GGCAATAGAGTAGGGCCAAAAAGCCTGACCTCACTCTAACTCAAAGTAATGTCCAGGTTCCCAGAGAATA
TCTGCTGGTATTTTTCTGTAAAGACCATTTGCAAAATTGTAACCTAATACAAAGTGTAGCCTTCTTCCAA
CTCAGGTAGAACACACCTGTCTTTGTCTTGCTGTTTTCACTCAGCCCTTTTAACATTTTCCCCTAAGCCC
ATATGTCTAAGGAAAGGATGCTATTTGGTAATGAGGAACTGTTATTTGTATGTGAATTAAAGTGCTCTTA
TTTTAAAAAATTGAAATAATTTTGATTTTTGCCTTCTGATTATTTAAAGATCTATATATGTTTTATTGGC
CCCTTCTTTATTTTAATAAAACAGTGAGAAATCTAAAAAAAAAAAAAAAAAA

>gi|153792494|ref|NM_004864.2| Homo sapiens growth differentiation factor 15 (GDF15), mRNA

AGTCCCAGCTCAGAGCCGCAACCTGCACAGCCATGCCCGGGCAAGAACTCAGGACGGTGAATGGCTCTCA
GATGCTCCTGGTGTTGCTGGTGCTCTCGTGGCTGCCGCATGGGGGCGCCCTGTCTCTGGCCGAGGCGAGC
CGCGCAAGTTTCCCGGGACCCTCAGAGTTGCACTCCGAAGACTCCAGATTCCGAGAGTTGCGGAAACGCT
ACGAGGACCTGCTAACCAGGCTGCGGGCCAACCAGAGCTGGGAAGATTCGAACACCGACCTCGTCCCGGC
CCCTGCAGTCCGGATACTCACGCCAGAAGTGCGGCTGGGATCCGGCGGCCACCTGCACCTGCGTATCTCT
CGGGCCGCCCTTCCCGAGGGGCTCCCCGAGGCCTCCCGCCTTCACCGGGCTCTGTTCCGGCTGTCCCCGA
CGGCGTCAAGGTCGTGGACGTGACACGACCGCTGCGGCGTCAGCTCAGCCTTGCAAGACCCCAGGCGCC
CGCGCTGCACCTGCGACTGTCGCCGCCGCCGTCGCAGTCGGACCAACTGCTGGCAGAATCTTCGTCCGCA
CGGCCCCAGCTGGAGTTGCACTTGCGGCCGCAAGCCGCCAGGGGGCGCCGCAGAGCGCGTGCGCGCAACG
GGGACCACTGTCCGCTCGGGCCCGGGCGTTGCTGCCGTCTGCACACGGTCCGCGCGTCGCTGGAAGACCT
GGGCTGGGCCGATTGGGTGCTGTCGCCACGGGAGGTGCAAGTGACCATGTGCATCGGCGCGTGCCCGAGC
CAGTTCCGGGCGGCAAACATGCACGCGCAGATCAAGACGAGCCTGCACCGCCTGAAGCCCGACACGGTGC
CAGCGCCCTGCTGCGTGCCCGCCAGCTACAATCCCATGGTGCTCATTCAAAAGACCGACACCGGGGTGTC
GCTCCAGACCTATGATGACTTGTTAGCCAAAGACTGCCACTGCATATGAGCAGTCCTGGTCCTTCCACTG
TGCACCTGCGCGGAGGACGCGACCTCAGTTGTCCTGCCCTGTGGAATGGGCTCAAGGTTCCTGAGACACC
CGATTCCTGCCCAAACAGCTGTATTTATATAAGTCTGTTATTTATTATTAATTTATTGGGGTGACCTTCT
TGGGGACTCGGGGGCTGGTCTGATGGAACTGTGTATTTATTTAAAACTCTGGTGATAAAAATAAAGCTGT
CTGAACTGTTAAAAAAAAAAAAAAAAAAAA

>gi|42542384|ref|NM_006928.3| Homo sapiens silver homolog (mouse) (SILV), mRNA

AGTGCCTTTGGTTGCTGGAGGGAAGAACACAATGGATCTGGTGCTAAAAAGATGCCTTCTTCATTTGGCT
GTGATAGGTGCTTTGCTGGCTGTGGGGGCTACAAAAGTACCCAGAAACCAGGACTGGCTTGGTGTCTCAA
GGCAACTCAGAACCAAAGCCTGGAACAGGCAGCTGTATCCAGAGTGGACAGAAGCCCAGAGACTTGACTG
CTGGAGAGGTGGTCAAGTGTCCCTCAAGGTCAGTAATGATGGGCCTACACTGATTGGTGCAAATGCCTCC
TTCTCTATTGCCTTGAACTTCCCTGGAAGCCAAAAGGTATTGCCAGATGGGCAGGTTATCTGGGTCAACA
ATACCATCATCAATGGGAGCCAGGTGTGGGGAGGACAGCCAGTGTATCCCCAGGAAACTGACGATGCCTG
CATCTTCCCTGATGGTGGACCTTGCCCATCTGGCTCTTGGTCTCAGAAGAGAAGCTTTGTTTATGTCTGG
AAGACCTGGGGCCAATACTGGCAAGTTCTAGGGGGCCCAGTGTCTGGGCTGAGCATTGGGACAGGCAGGG
CAATGCTGGGCACACACACCATGGAAGTGACTGTCTACCATCGCCGGGGATCCCGGAGCTATGTGCCTCT
TGCTCATTCCAGCTCAGCCTTCACCATTACTGACCAGGTGCCTTTCTCCGTGAGCGTGTCCCAGTTGCGG
GCCTTGGATGGAGGGAACAAGCACTTCCTGAGAAATCAGCCTCTGACCTTTGCCCTCCAGCTCCATGACC
CCAGTGGCTATCTGGCTGAAGCTGACCTCTCCTACACCTGGGACTTTGGAGACAGTAGTGGAACCCTGAT
CTCTCGGGCACTTGTGGTCACTCATACTTACCTGGAGCCTGGCCCAGTCACTGCCCAGGTGGTCCTGCAG
GCTGCCATTCCTCTCACCTCCTGTGGCTCCTCCCCAGTTCCAGGCACCACAGATGGGCACAGGCCAACTG
CAGAGGCCCCTAACACCACAGCTGGCCAAGTGCCTACTACAGAAGTTGTGGGTACTACACCTGGTCAGGC
GCCAACTGCAGAGCCCTCTGGAACCACATCTGTGCAGGTGCCAACCACTGAAGTCATAAGCACTGCACCT
GTGCAGATGCCAACTGCAGAGAGCACAGGTATGACACCTGAGAAGGTGCCAGTTTCAGAGGTCATGGGTA
CCACACTGGCAGAGATGTCAACTCCAGAGGCTACAGGTATGACACCTGCAGAGGTATCAATTGTGGTGCT
TTCTGGAACCACAGCTGCACAGGTAACAACTACAGAGTGGGTGGAGACCACAGCTAGAGAGCTACCTATC
CCTGAGCCTGAAGGTCCAGATGCCAGCTCAATCATGTCTACGGAAAGTATTACAGGTTCCCTGGCCCCC
TGCTGGATGGTACAGCCACCTTAAGGCTGGTGAAGAGACAAGTCCCCCTGGATTGTGTTCTGTATCGATA
TGGTTCCTTTTCCGTCACCCTGGACATTGTCCAGGGTATTGAAAGTGCCGAGATCCTGCAGGCTGTGCCG
TCCGGTGAGGGGGATGCATTTGAGCTGACTGTGTCCTGCCAAGGCGGGCTGCCCAAGGAAGCCTGCATGG
AGATCTCATCGCCAGGGTGCCAGCCCCCTGCCCAGCGGCTGTGCCAGCCTGTGCTACCCAGCCCAGCCTG
CCAGCTGGTTCTGCACCAGATACTGAAGGGTGGCTCGGGGACATACTGCCTCAATGTGTCTCTGGCTGAT
ACCAACAGCCTGGCAGTGGTCAGCACCCAGCTTATCATGCCTGGTCAAGAAGCAGGCCTTGGGCAGGTTC
CGCTGATCGTGGGCATCTTGCTGGTGTTGATGGCTGTGGTCCTTGCATCTCTGATATATAGGCGCAGACT
TATGAAGCAAGACTTCTCCGTACCCCAGTTGCCACATAGCAGCAGTCACTGGCTGCGTCTACCCCGCATC
TTCTGCTCTTGTCCCATTGGTGAGAATAGCCCCCTCCTCAGTGGGCAGCAGGTCTGAGTACTCTCATATG
ATGCTGTGATTTTCCTGGAGTTGACAGAAACACCTATATTTCCCCCAGTCTTCCCTGGGAGACTACTATT
AACTGAAATAAATACTCAGAGCCTGAAAAAAAAAAAAAAAAAA

>gi|13435354|ref|NM_000425.2| Homo sapiens L1 cell adhesion molecule (L1CAM), transcript variant 1, mRNA

GCGCGGTGCCGCCGGGAAAGATGGTCGTGGCGCTGCGGTACGTGTGGCCTCTCCTCCTCTGCAGCCCCTG
CCTGCTTATCCAGATCCCCGAGGAATATGAAGGACACCATGTGATGGAGCCACCTGTCATCACGGAACAG
TCTCCACGGCGCCTGGTTGTCTTCCCCACAGATGACATCAGCCTCAAGTGTGAGGCCAGTGGCAAGCCCG
AAGTGCAGTTCCGCTGGACGAGGGATGGTGTCCACTTCAAACCCAAGGAAGAGCTGGGTGTGACCGTGTA
CCAGTCGCCCCACTCTGGCTCCTTCACCATCACGGGCAACAACAGCAACTTTGCTCAGAGGTTCCAGGGC
ATCTACCGCTGCTTTGCCAGCAATAAGCTGGGCACCGCCATGTCCCATGAGATCCGGCTCATGGCCGAGG
GTGCCCCCAAGTGGCCAAAGGAGACAGTGAAGCCCGTGGAGGTGGAGGAAGGGGAGTCAGTGGTTCTGCC
TTGCAACCCTCCCCCAAGTGCAGAGCCTCTCCGGATCTACTGGATGAACAGCAAGATCTTGCACATCAAG
CAGGACGAGCGGGTGACGATGGGCCAGAACGGCAACCTCTACTTTGCCAATGTGCTCACCTCCGACAACC
ACTCAGACTACATCTGCCACGCCCACTTCCCAGGCACCAGGACCATCATTCAGAAGGAACCCATTGACCT
CCGGGTCAAGGCCACCAACAGCATGATTGACAGGAAGCCGCGCCTGCTCTTCCCCACCAACTCCAGCAGC
CACCTGGTGGCCTTGCAGGGGCAGCCATTGGTCCTGGAGTGCATCGCCGAGGGCTTTCCCACGCCCACCA
TCAAATGGCTGCGCCCCAGTGGCCCCATGCCAGCCGACCGTGTCACCTACCAGAACCACAACAAGACCCT
GCAGCTGCTGAAAGTGGGCGAGGAGGATGATGGCGAGTACCGCTGCCTGGCCGAGAACTCACTGGGCAGT
GCCCGGCATGCGTACTATGTCACCGTGGAGGCTGCCCCGTACTGGCTGCACAAGCCCCAGAGCCATCTAT
ATGGGCCAGGAGAGACTGCCCGCCTGGACTGCCAAGTCCAGGGCAGGCCCCAACCAGAGGTCACCTGGAG
AATCAACGGGATCCCTGTGGAGGAGCTGGCCAAAGACCAGAAGTACCGGATTCAGCGTGGCGCCCTGATC
CTGAGCAACGTGCAGCCCAGTGACACAATGGTGACCCAATGTGAGGCCCGCAACCGGCACGGGCTCTTGC
TGGCCAATGCCTACATCTACGTTGTCCAGCTGCCAGCCAAGATCCTGACTGCGGACAATCAGACGTACAT
GGCTGTCCAGGGCAGCACTGCCTACCTTCTGTGCAAGGCCTTCGGAGCGCCTGTGCCCAGTGTTCAGTGG
CTGGACGAGGATGGGACAACAGTGCTTCAGGACGAACGCTTCTTCCCCTATGCCAATGGGACCCTGGGCA
TTCGAGACCTCCAGGCCAATGACACCGGACGCTACTTCTGCCTGGCTGCCAATGACCAAAACAATGTTAC
CATCATGGCTAACCTGAAGGTTAAAGATGCAACTCAGATCACTCAGGGGCCCCGCAGCACAATCGAGAAG
AAAGGTTCCAGGGTGACCTTCACGTGCCAGGCCTCCTTTGACCCCTCCTTGCAGCCCAGCATCACCTGGC
GTGGGGACGGTCGAGACCTCCAGGAGCTTGGGGACAGTGACAAGTACTTCATAGAGGATGGGCGCCTGGT
CATCCACAGCCTGGACTACAGCGACCAGGGCAACTACAGCTGCGTGGCCAGTACCGAACTGGATGTGGTG
GAGAGTAGGGCACAGCTCTTGGTGGTGGGGAGCCCTGGGCCGGTGCCACGGCTGGTGCTGTCCGACCTGC
ACCTGCTGACGCAGAGCCAGGTGCGCGTGTCCTGGAGTCCTGCAGAAGACCACAATGCCCCCATTGAGAA

ATATGACATTGAATTTGAGGACAAGGAAATGGCGCCTGAAAAATGGTACAGTCTGGGCAAGGTTCCAGGG
AACCAGACCTCTACCACCCTCAAGCTGTCGCCCTATGTCCACTACACCTTTAGGGTTACTGCCATAAACA
AATATGGCCCCGGGGAGCCCAGCCCGGTCTCTGAGACTGTGGTCACACCTGAGGCAGCCCCAGAGAAGAA
CCCTGTGGATGTGAAGGGGGAAGGAAATGAGACCACCAATATGGTCATCACGTGGAAGCCGCTCCGGTGG
ATGGACTGGAACGCCCCCCAGGTTCAGTACCGCGTGCAGTGGCGCCCTCAGGGGACACGAGGGCCCTGGC
AGGAGCAGATTGTCAGCGACCCCTTCCTGGTGGTGTCCAACACGTCCACCTTCGTGCCCTATGAGATCAA
AGTCCAGGCCGTCAACAGCCAGGGCAAGGGACCAGAGCCCCAGGTCACTATCGGCTACTCTGGAGAGGAC
TACCCCCAGGCAATCCCTGAGCTGGAAGGCATTGAAATCCTCAACTCAAGTGCCGTGCTGGTCAAGTGGC
GGCCGGTGGACCTGGCCCAGGTCAAGGGCCACCTCCGCGGATACAATGTGACGTACTGGAGGGAGGGCAG
TCAGAGGAAGCACAGCAAGAGACATATCCACAAAGACCATGTGGTGGTGCCCGCCAACACCACCAGTGTC
ATCCTCAGTGGCTTGCGGCCCTATAGCTCCTACCACCTGGAGGTGCAGGCCTTTAACGGGCGAGGATCGG
GGCCCGCCAGCGAGTTCACCTTCAGCACCCCAGAGGGAGTGCCTGGCCACCCCGAGGCGTTGCACCTGGA
GTGCCAGTCGAACACCAGCCTGCTGCTGCGCTGGCAGCCCCCACTCAGCCACAACGGCGTGCTCACCGGC
TACGTGCTCTCCTACCACCCCCTGGATGAGGGGGGCAAGGGGCAACTGTCCTTCAACCTTCGGGACCCCG
AACTTCGGACACACAACCTGACCGATCTCAGCCCCCACCTGCGGTACCGCTTCCAGCTTCAGGCCACCAC
CAAAGAGGGCCCTGGTGAAGCCATCGTACGGGAAGGAGGCACTATGGCCTTGTCTGGGATCTCAGATTTT
GGCAACATCTCAGCCACAGCGGGTGAAAACTACAGTGTCGTCTCCTGGGTCCCCAAGGAGGGCCAGTGCA
ACTTCAGGTTCCATATCTTGTTCAAAGCCTTGGGAGAAGAGAAGGGTGGGGCTTCCCTTTCGCCACAGTA
TGTCAGCTACAACCAGAGCTCCTACACGCAGTGGGACCTGCAGCCTGACACTGACTACGAGATCCACTTG
TTTAAGGAGAGGATGTTCCGGCACCAAATGGCTGTGAAGACCAATGGCACAGGCCGCGTGAGGCTCCCTC
CTGCTGGCTTCGCCACTGAGGGCTGGTTCATCGGCTTTGTGAGTGCCATCATCCTCCTGCTCCTCGTCCT
GCTCATCCTCTGCTTCATCAAGCGCAGCAAGGGCGGCAAATACTCAGTGAAGGATAAGGAGGACACCCAG
GTGGACTCTGAGGCCCGACCGATGAAAGATGAGACCTTCGGCGAGTACAGGTCCCTGGAGAGTGACAACG
AGGAGAAGGCCTTTGGCAGCAGCCAGCCATCGCTCAACGGGGACATCAAGCCCCTGGGCAGTGACGACAG
CCTGGCCGATTATGGGGGCAGCGTGGATGTTCAGTTCAACGAGGATGGTTCGTTCATTGGCCAGTACAGT
GGCAAGAAGGAGAAGGAGGCGGCAGGGGGCAATGACAGCTCAGGGGCCACTTCCCCCATCAACCCTGCCG
TGGCCCTAGAATAGTGGAGTCCAGGACAGGAGATGCTGTGCCCCTGGCCTTGGGATCCAGGCCCCTCCCT
CTCCAGCAGGCCCATGGGAGGCTGGAGTTGGGGCAGAGGAGAACTTGCTGCCTCGGATCCCCTTCCTACC
ACCCGGTCCCCACTTTATTGCCAAAACCCAGCTGCACCCCTTCCTGGGCACACGCTGCTCTGCCCCAGCT
TGGGCAGATCTCCCACATGCCAGGGGCCTTTGGGTGCTGTTTTGCCAGCCCATTTGGGCAGAGAGGCTGT
GGTTTGGGGGAGAAGAAGTAGGGGTGGCCCGAAAGGGTCTCCGAAATGCTGTCTTTCTTGCTCCCTGACT
GGGGGCAGACATGGTGGGGTCTCCTCAGGACCAGGGTTGGCACCTTCCCCCTCCCCCAGCCACTCCCCAG
CCAGCCTGGCTGGGACTGGGAACAGAACTCGGTGTCCCCACCATCTGCTGTCTTTTCTTTGCCATCTCTG
CTCCAACCGGGATGGGAGCCGGGCAAACTGGCCGCGGGGGCAGGGGAGGCCATCTGGAGAGCCCAGAGTC
CCCCCACTCCCAGCATCGCACTCTGGCAGCACCGCCTCTTCCCGCCGCCCAGCCCACCCCATGGCCGGCT
TTCAGGAGCTCCATACACACGCTGCCTTCGGTACCCACCACACAACATCCAAGTGGCCTCCGTCACTACC
TGGCTGCGGGGCGGGCACACCTCCTCCCACTGCCCACTGGCCGGC

## Claims

1. A method of identifying a melanoma in a tissue sample comprising the steps of

   a) measuring the expression levels in the sample of genes encoding mRNA corresponding to SILV;
   b) measuring the expression level of a gene encoding tyrosinase; and
   c) measuring the expression level of SILV relative to tyrosinase;

   wherein the expression levels of SILV relative to tyrosinase are indicative of the presence of a melanoma.

2. The method of claim 1 wherein the sample is a primary skin biopsy sample.

3. The method of claim 1 or claim 2, wherein gene expression is measured on a microarray or genechip.

4. The method of claim 1 or claim 2 wherein gene expression is determined by nucleic acid amplification conducted by polymerase chain reaction (PCR) of RNA extracted from the sample.

5. The method of claim 4 wherein the polymerase chain reaction is qRT-PCR.

6. The use of a kit in the method of any one of claim 1 to 4, wherein the kit comprises nucleic acid amplification and detection reagents.

7. The use of claim 6 wherein the nucleic acid amplification reagents comprise primers with the sequence of SEQ ID NOs 1 and 2 and a probe with the sequence of SEQ ID NO: 3.

## Patentansprüche

1. Verfahren zum Identifizieren eines Melanoms in einer Gewebeprobe, umfassend die folgenden Schritte:

   a) Messen der Expressionsniveaus von Genen, die für mRNA codieren, entsprechend SILV, in der Probe;
   b) Messen des Expressionsniveaus eines Gens, das für Tyrosinase codiert; und
   c) Messen des Expressionsniveaus von SILV im Vergleich zu Tyrosinase;

   wobei die Expressionsniveaus von SILV im Vergleich zu Tyrosinase das Vorliegen eines Melanoms anzeigen.

2. Verfahren nach Anspruch 1, wobei es sich bei der Probe um eine primäre Hautbiopsieprobe handelt.

3. Verfahren nach Anspruch 1 oder Anspruch 2, wobei die Genexpression auf einem Mikro-Array oder Gen-Chip gemessen wird.

4. Verfahren nach Anspruch 1 oder Anspruch 2, wobei die Genexpression mittels Nukleinsäureamplifikation, die durch Polymerasekettenreaktion (PCR) von aus der Probe extrahierter RNA durchgeführt wird.

5. Verfahren nach Anspruch 4, wobei es sich bei der Polymerasekettenreaktion um qRT-PCR handelt.

6. Verwendung eines Kits in dem Verfahren nach einem der Ansprüche 1 bis 4, wobei das Kit Nukleinsäureamplifikations- und -nachweisreagenzien umfasst.

7. Verwendung nach Anspruch 6, wobei die Nukleinsäureamplifikationsreagenzien Primer mit der Sequenz von SEQ ID NO: 1 und 2 und eine Sonde mit der Sequenz von SEQ ID NO: 3 umfassen.

## Revendications

1. Procédé d'identification d'un mélanome dans un échantillon tissulaire, comprenant les étapes consistant à

a) mesurer les niveaux d'expression dans l'échantillon de gènes codant pour un ARNm correspondant à une SILV ;
b) mesurer le niveau d'expression d'un gène codant pour une tyrosinase ; et
c) mesurer le niveau d'expression de SILV par rapport à une tyrosinase ;

dans lequel les niveaux d'expression de SILV par rapport à une tyrosinase sont indicatifs de la présence d'un mélanome.

2. Procédé selon la revendication 1, dans laquelle l'échantillon est un échantillon de biopsie cutanée primaire.

3. Procédé selon la revendication 1 ou la revendication 2, dans laquelle l'expression génique est mesurée sur un micro-arrangement ou une puce à ADN.

4. Procédé selon la revendication 1 ou la revendication 2, dans laquelle l'expression génique est déterminée par une amplification d'acide nucléique conduite par une réaction en chaîne par polymérase (ACP) d'un ARN extrait de l'échantillon.

5. Procédé selon la revendication 4, dans laquelle la réaction en chaîne par polymérase est une ACPq-TI.

6. Utilisation d'une trousse dans le procédé selon l'une quelconque des revendications 1 à 4, dans laquelle la trousse comprend des réactifs pour une amplification et une détection d'acides nucléiques.

7. Utilisation selon la revendication 6, dans laquelle les réactifs pour une amplification d'acides nucléiques comprennent des amorces, avec la séquence des SEQ ID n° 1 et 2, ainsi qu'une sonde avec la séquence de SEQ ID n° : 3.

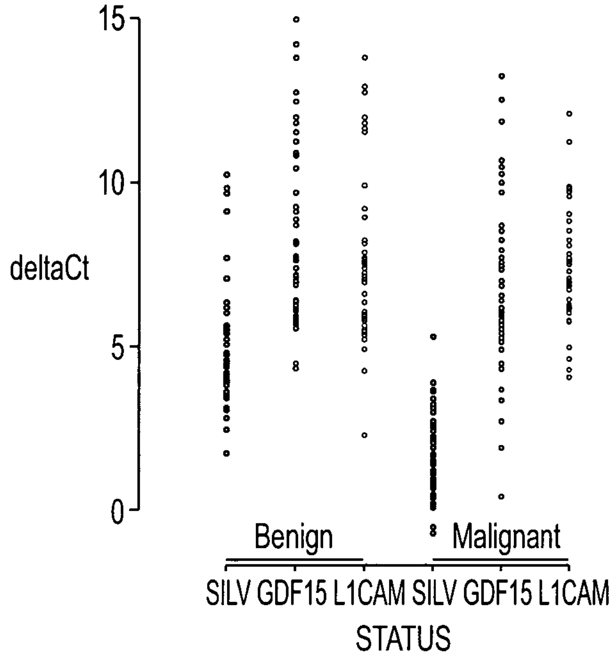
15
10
5
0
deltaCt
Benign
Malignant
SILV GDF15 L1CAM SILV GDF15 L1CAM
STATUS

*FIG. 1*

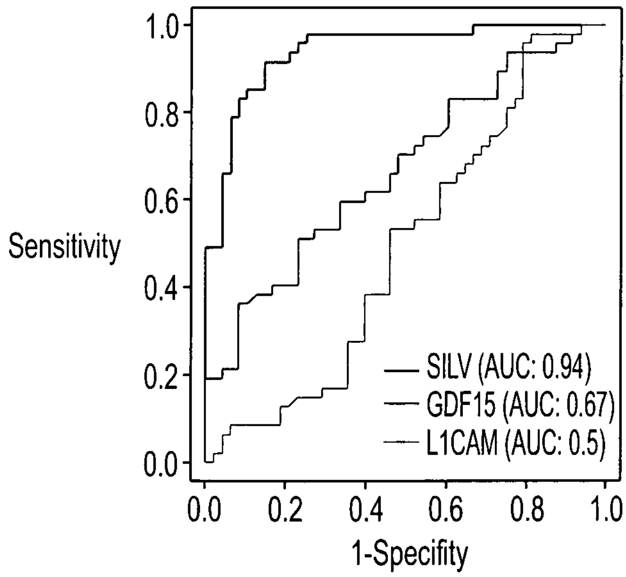
1.0
0.8
0.6
0.4
0.2
0.0
Sensitivity
SILV (AUC: 0.94)
GDF15 (AUC: 0.67)
L1CAM (AUC: 0.5)
0.0 0.2 0.4 0.6 0.8 1.0
1-Specifity

*FIG. 2*

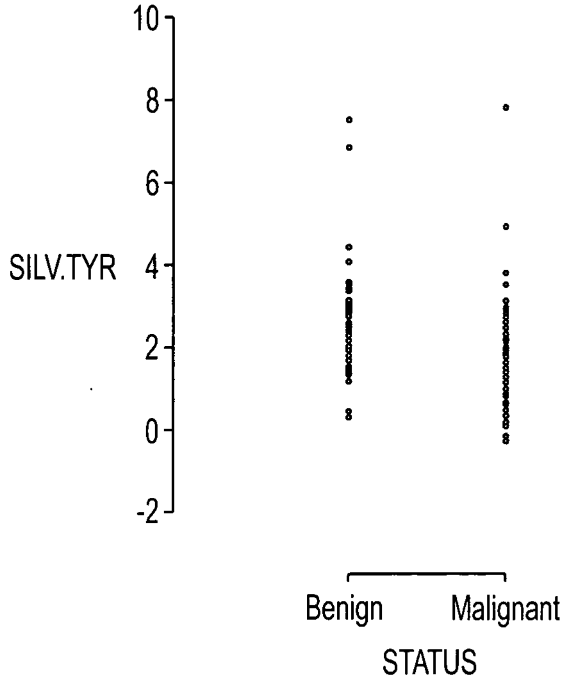
10
8
6
SILV.TYR 4
2
0
-2
Benign
Malignant
STATUS

*FIG. 3*

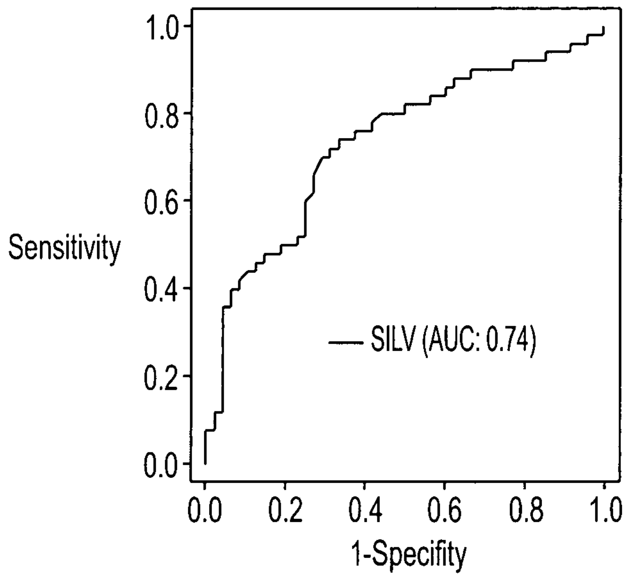
1.0
0.8
0.6
Sensitivity
0.4
0.2
0.0
— SILV (AUC: 0.74)
0.0 0.2 0.4 0.6 0.8 1.0
1-Specifity

*FIG. 4*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006002433 A
- US 20070154889 A
- US 5411876 A
- US 5985619 A
- US 5550044 A
- US 5413924 A
- US 6403341 B
- US 6153388 A
- US 5210015 A
- US 5487972 A
- US 5804375 A
- US 61223894 B